# EUROPEAN PATENT SPECIFICATION

(11) **EP 1 981 354 B1**
(45) Date of publication and mention of the grant of the patent: **15.11.2017**
(21) Application number: 07717323.5
(22) Date of filing: 19.01.2007
(51) Int. Cl.: A23K 10/30, A23K 20/147, A23K 20/22, A23K 20/26, A23K 50/40

(54) **COMPOSITIONS AND METHODS FOR PREVENTING FELINE CHRONIC KIDNEY DISEASE**
ZUSAMMENSETZUNGEN UND VERFAHREN FÜR DIE VORBEUGUNG VON CHRONISCHER NIERENERKRANKUNG BEI KATZEN
COMPOSITIONS ET PROCEDES DESTINES A PREVENIR UNE MALADIE RENALE CHRONIQUE CHEZ LES FELINS

(30) Priority: 19.01.2006 US 760068 P
(43) Date of publication of application: 22.10.2008
(60) Divisional application: 12158853.7
(73) Proprietor: Hill's Pet Nutrition, Inc., Topeka, KS 66603 (US)
(72) Inventor: WEDEKIND, Karen, Joy, St. Peters MO 63376 (US)
(74) Representative: Wichmann, Hendrik
(86) International application number: PCT/US2007/060773
(87) International publication number: WO 2007/084986

(56) References cited:
- WO-A1-2007/133726
- US-A- 6 039 952
- US-A1- 2005 064 016
- ANONYMOUS: "Ami Products - Ingredients" INTERNET ARTICLE, [Online] 24 June 2004 (2004-06-24), XP002446899 Retrieved from the Internet: URL:http://web.archive.org/web/20030624115 116/http://ami.aminews.net/en_ingredienti. html> [retrieved on 2007-08-14]
- ANONYMOUS: "Benevo Vegetarian Cat Food" INTERNET ARTICLE, [Online] 10 September 2005 (2005-09-10), XP002446900 Retrieved from the Internet: URL:http://web.archive.org/web/20050910051 942/http://www.veggiepets.com/acatalog/ben evo_vegetarian_cat_food.html> [retrieved on 2007-08-14]
- HUGHES K L; SLATER M R; GELLER S; BURKHOLDER W J; FITZGERALD C: "Diet and lifestyle variables as risk factors for chronic renal failure in pet cats" PREVENTIVE VETERINARY MEDICINE, vol. 55, no. 1, 10 September 2002 (2002-09-10), pages 1-15, XP002446901
- BROOKS, WENDY C: "Kidney Failure: Where to Begin" INTERNET ARTICLE, [Online] 1 January 2001 (2001-01-01), XP002446903 Retrieved from the Internet: URL:http://www.veterinarypartner.com/Conte nt.plx?P=A&S=0&C=0&A=572> [retrieved on 2007-08-15]
- POLZIN D J; OSBORNE C A; ROSS S; JACOB F: "Dietary management of feline chronic renal failure: where are we now? In what direction are we headed?" JOURNAL OF FELINE MEDICINE AND SURGERY, June 2000 (2000-06), pages 75-82, XP002446902
- BROWN S A ET AL: "Pathophysiology and management of progressive renaldisease" VETERINARY JOURNAL, BAILLIERE TINDALL, LONDON,, GB, vol. 154, no. 2, September 1997 (1997-09), pages 93-109, XP004762583 ISSN: 1090-0233

## Description

This application claims benefit of U.S. Provisional No. 60/760,068 filed January 19, 2006.

### BACKGROUND OF THE INVENTION

### Field of the Invention

The invention relates generally to compositions for use in preventing feline chronic kidney disease.

### Description of the Related Art

The kidneys have five primary functions. They filter waste products from the body (e.g., urea and creatinine), regulate electrolytes (e.g., potassium, calcium, phosphorus, and sodium), produce erythropoietin (which stimulates bone marrow to produce red blood cells), produce rennin (which controls blood pressure), and produce and concentrate urine.

Chronic kidney disease ("CKD"), e.g., chronic renal failure, is a progressive kidney disease which has four phases: loss of renal reserve, renal insufficiency, azotemia, and uremia. The kidneys have a large built-in reserve as only approximately 30% of kidney capacity is needed for normal kidney function. Kidney capacity diminishes with time for a variety of reasons, for example, advanced age and diseases and medications that damage the kidney(s). Renal insufficiency is characterized by decrease in renal function, and is generally observed when about 70% of kidney function has been lost *(i.e.,* when only about 30% of kidney capacity is available). Clinical signs are typically not obvious during the phases of loss of renal reserve and renal insufficiency, thereby making it difficult to detect CKD. Azotemia, the third phase of CKD, is characterized by an abnormally high concentration of urea, creatinine, and/or other non-protein nitrogen-containing substances in the blood. Renal function is markedly reduced in the azotemic phase, although clinical signs may be mild and can go undetected. Uremia is characterized by the presence of abnormal quantities of urine or urine constituents in blood. The uremic phase is characterized by overt clinical signs

CKD is a terminal disease. It is one of the leading causes of death in felines. Thus, there is a need for compositions and methods for preventing CKD in felines. There is also a need for compositions and methods for treating CKD which provide partial or complete relief.

Monitoring creatinine is useful to measure renal function. For example, as a renal disease progresses, creatinine levels increase in blood serum. It is believed an increase creatinine serum levels is associated with a decrease in glomerular filtration rate. Therefore, a food composition or diet that prevents an increase, or causes a decrease in serum creatinine levels would have a beneficial effect in felines predisposed to or suffering from CKD.

ANONYMOUS: "Ami Products - Ingredients " INTERNET ARTICLE, [Online] 24 June 2004 [2004-06-24], XP002446899 Retrieved from the Internet: URL:http://web.archive.org/web/20030624115116/http://ami.aminews.net/en_ingredienti.htm 1 [retrieved on 2007-08-14] discloses a cat food composition comprising 36% protein, 0.93% phosphorus, 0.35% sodium and 0.19% potassium on a dry matter basis.

ANONYMOUS: "Benevo Vegetarian Cat Food" INTERNET ARTICLE, [Online] 10 September 2005 [2005-09-10], XP002446900 Retrieved from the Internet: URL:http://web.archive.org/web/20050910051942/http://www.veggiepets.com/ actatalog/benevo_vegetarian_cat_food.html [retrieved on 2007-08-14] discloses a cat food composition comprising 28% protein, wherein 100% of the protein is vegetable protein.

US 6039952 discloses a pet food composition comprising from about 10 to about 32% crude protein and optionally less than about 0.50% phosphorus for use in improving clinical signs associated with renal disease in companion animals.

US 2005/064016 A1 discloses a cat food composition comprising 30-40% protein for use in reducing risk of developing hyperthyroidism in a feline.

BROOKS, WENDY C: "Kidney Failure: Where to Begin" INTERNET ARTICLE, [Online] 1 January 2001 [2001-01-01], XP002446903 Retrieved from the Internet: URL:htt://www.veterinary partner.com/Content.plx?P=A&S=0&C=0&A=572 [retrieved on 2007-08-15] discloses biological indicators of kidney function in pets.

### SUMMARY OF THE INVENTION

One object of the invention is to provide compositions for use in preventing CKD in felines. Another object is to provide the use of such a composition to prepare a medicament for use in preventing CKD in felines.

Accordingly, the present invention provides a composition for use according to claim 1.

The present invention also provides a use according to claim 5.

Additional objects, features, and advantages of the invention will be apparent to those skilled in the art.

The invention is defined by the claims. Methods, compositions and uses falling outside the scope of the claims are not according to the invention and are provided for comparison only.

### DETAILED DESCRIPTION

In one aspect, the present disclosure provides a method for preventing CKD in a feline susceptible to developing CKD. The method comprises feeding the feline a composition comprising from about 33 to about 38%, or from about 28 to about 35% protein on a dry matter basis wherein the protein comprises at least about 75% vegetable protein. In some embodiments, the method comprises feeding the feline a composition that comprises from about 33 to about 35%, or from about 28 to about 31% protein wherein the protein comprises at least about 75% vegetable protein. In some such embodiments, the method comprises feeding the feline a composition that comprises from about 33 to about 34%, or from about 28 to about 30% protein. In other such embodiments, the method comprises feeding the feline a composition that comprises from about 34 to about 35%, or from about 29 to about 31 % protein wherein the protein comprises at least about 75, at least about 80, at least about 85, at least about 90, or at least about 95% vegetable protein. In yet other such embodiments, the method comprises feeding the feline a composition that comprises from about 28 or about 28.5 to about 29. about 29.5, about 30, about 30.5, or about 31% protein wherein the protein comprises at least about 75. at least about 80, at least about 85 at least about 90, or at least about 95% vegetable protein. In another embodiment, the method comprises feeding the feline a composition that comprises from about 33 or about 33.5 to about 34. about 34.5, or about 35% protein wherein the protein comprises at least about 75, at least about 80. at least about 85. at least about 90, or at least about 95% vegetable protein. In some embodiments, the method comprises feeding the feline a composition selected from the compositions of the invention for preventing renal insufficiency. One skilled in the art would understand that either a single composition of the invention can be fed to the feline or, alternatively, different such compositions can be fed to the feline for varying time intervals.

In other such embodiments, the method comprises feeding the feline a composition comprising from about 31 to about 35%, or from about 35 to about 38% protein wherein the protein comprises at least about 75% vegetable protein. In some such embodiments, the method comprises feeding the feline a composition comprising from about 32 to about 34%, or from about 36 to about 37% protein wherein the protein comprises at least about 75% vegetable protein. In other such embodiments, the method comprises feeding the feline a composition comprising from about 32 to about 35%, or from about 36 to about 38% protein wherein the protein comprises at least about 75% vegetable protein. In yet other such embodiments, the method comprises feeding the feline a composition comprising from about 33 to about 35%, or from about 36.5 to about 38% protein wherein the protein comprises at least about 75% vegetable protein. In further such embodiments. the method comprises feeding the feline a composition comprising from about 31, about 32, or about 33 to about 34 or about 35% protein wherein the protein comprises at least about 75, at least about 80, at least about 85, at least about 90, or at least about 95% vegetable protein. In another embodiment, the method comprises feeding the feline a composition comprising from about 35, about 36, or about 36.5 to about 37 or about 38% protein wherein the protein comprises at least about 75, at least about 80, at least about 85%, at least about 90, or at least about 95% vegetable protein. In another embodiment, the method comprises feeding the feline a composition comprising from about 31 to about 32, about 33. about 34, or about 35% protein wherein the protein comprises at least about 75, at least about 80. at least about 85, at least about 90, or at least about 95% vegetable protein. In another embodiment, the method comprises feeding the feline a composition comprising from about 35 to about 36, about 36.5, about 37, or about 38% protein wherein the protein comprises at least about 75, at least about 80. at least about 85, at least about 90, or at least about 95% vegetable protein.

In some embodiments of the method for preventing CKD, the composition that is fed to the feline further comprises from about 0.45 to about 0.8%. or from 0.51 to about 0.70% phosphorus on a dry matter basis. In some such embodiments, the composition that is fed to the feline comprises from about 0.45 to about 0.6%, or from about .51 to about .65% phosphorus, for example, from about 0.45 to about 0.55%, from about 0.45 to about 0.5% phosphorus, from about 0.51 to about 0.60% or from about 0.51 to about 0.55% phosphorus. In other such embodiments, the composition fed to the feline comprises from about 0.6 to about 0.8% phosphorus, for example, from about 0.7 to about 0.8% or from about 0.6 or 0.65 to about 0.75% phosphorus.

In some embodiments of the method for preventing CKD, the composition that is fed to the feline further comprises from about 0.2 or 0.25 to about 0.40%, or from about 0.2 to about 0.35% sodium on a dry matter basis. In some such embodiments, the composition fed to the feline comprises from about 0.2 to about 0.35% sodium, and in other such embodiments, the composition fed to the feline comprises from about 0.2 to about 0.25% sodium.

In some embodiments of the method for preventing CKD, the composition that is fed to the feline further comprises from about 0.75 to about 1% potassium on a dry matter basis. In some such embodiments, the composition fed to the feline comprises from about 0.8 to about 1% potassium, and in other such embodiments, the composition comprises from about 0.75 to about 0.9% potassium.

In some embodiments of the method for preventing CKD, the composition fed to the feline further comprises two or more of phosphorus, sodium, and potassium in any combination of the amounts discussed above.

In another aspect, the present disclosure provides a method for treating CKD in a feline. The method comprises feeding the feline a composition comprising from about 26 to about 30%, or about 28 to about 30% protein on a dry matter basis wherein the protein present in the composition comprises at least about 75% vegetable protein. In some embodiments, the method comprises feeding the feline a composition comprising from about 26 to about 27%, or from about 28 to about 29% protein wherein the protein comprises at least about 75% vegetable protein. In other such embodiments, the method comprises feeding the feline a composition comprising from about 27 or 29 to about 30% protein wherein the protein comprises at least about 75% vegetable protein. In some embodiments, the method comprises feeding the feline a composition comprising from about 28 or about 28.5 to about 29, about 29.5, or about 30% protein wherein the protein comprises at least about 75, at least about 80, at least about 85, at least about 90, or at least about 95% vegetable protein. In some embodiments, the composition comprises from about 26 or about 27 to about 28, about 29, or about 30% protein wherein the protein comprises at least about 75, at least about 80, at least about 85, at least about 90, or at least about 95% vegetable protein. In some embodiments, the method comprises feeding the feline a composition selected from the compositions of the present disclosure for treating CKD. One skilled in the art would understand that either a single composition of the disclosure can be fed to the feline or, alternatively, different such compositions can be fed to the feline for varying time intervals.

In some embodiments of the method for treating CKD in a feline, the composition that is fed to the feline further comprises from about 0.20 to about 0.50%, or from about 0.45 to about 0.6% phosphorus on a dry matter basis. In some such embodiments, the composition fed to the feline comprises from about 0.20 to about .045%, or from about 0.45 to about 0.55% phosphorus. In other such embodiments, the composition fed to the feline comprises from about 0.20 to about 0.40%, or from about 0.45 to about 0.5% phosphorus.

In some embodiments of the method for treating CKD in a feline, the composition that is fed to the feline further comprises from about 0.2 to about 0.25%, or from about 0.23 to about 0.30% sodium on a dry matter basis.

In some embodiments of the method for treating CKD in a feline, the composition that is fed to the feline further comprises from about 0.75 to about 1% potassium on a dry matter basis. In some such embodiments, the composition that is fed to the feline comprises from about 0.7 to about 0.9% potassium. In other such embodiments, the composition fed to the feline comprises from about 0.75 to about 0.85% potassium.

In some embodiments of the method for treating CKD in a feline, the composition fed to the feline further comprises two or more of phosphorus, sodium, and potassium in any combination of the amounts discussed above.

In some embodiments, the methods of the disclosure for prevention and treatment of CKD further comprise administering to the feline an anti-CKD agent. An anti-CKD agent is a compound, a derivative thereof (*e.g*., a salt, solvate, or hydrate of the compound), or a composition comprising such compounds and/or derivatives that is used to prevent and/or treat CKD. Such an agent is administered in conjunction with feeding a composition of the invention. The term "in conjunction" means that an agent is administered to the feline either together with a composition of the present invention or separately from the composition at the same or different frequency via the same or different administration route and either at about the same time as the composition or periodically. "Administering" means that the agent is introduced in a suitable dosage form into the feline by a suitable administration route, for example, orally, topically, or parenterally. "About at the same time" generally means that an agent is administered when a composition of the invention is fed to the feline or within about 72 hours of feeding the composition to the feline. "Periodically" generally means that an agent is administered to a feline following a dosage schedule suitable for administering the agent while a composition of the invention is fed to the feline routinely as appropriate for that feline. Thus, the term "in conjunction" specifically includes situations when an agent is administered to a feline for a prescribed period of time while a composition of the invention is fed to the feline for a much longer period of time (e.g., for life). If more than one agent is administered, the dosage form and route of administration for each agent may vary. In addition, one composition of the invention may be substituted with another composition of the invention while a specific agent is administered to the feline.

Suitable anti-CKD agents include, for example, agents that reduce blood pressure, e.g., calcium channel blockers (e.g., amlodipine besylate) or protein loss in urine, ACE-inhibitors (e.g., benazepril), anabolic steroids, antibiotics, agents that treat anemia, e.g., feline recombinant erythropoietin, diuretics, e.g., lasix furoesemide, and agents that control vomiting, e.g., anti-emetics (e.g., REGLAN®) Such agents can be administered, for example, in the form of salts derived from inorganic or organic acids. Depending on the particular compound, a salt of the compound may be advantageous due to one or more of the salt's physical properties, for example, enhanced pharmaceutical stability in differing temperatures and humidities, or a desirable solubility in water or oil. The salt preferably is a pharmaceutically-acceptable salt.

The total daily dose of an anti-CKD agent (administered in single or divided doses) is typically from about 0.001 to about 100 mg/kg, preferably from about 0.001 to about 30 mg/kg, and even more preferably from about 0.01 to about 10 mg/kg body weight. Dosage unit compositions can contain such amounts or submultiples thereof to make up the daily dose. In many instances, the administration of the anti-CKD agent will be repeated a plurality of times. Multiple doses per day typically may be used to increase the total daily dose. Factors affecting the preferred dosage regimen include, for example, the age, weight, and condition of the feline; the severity of the disease; the route of administration; pharmacological considerations (e*.g.,* activity, efficacy, and pharmacokinetic and toxicology profiles of the particular anti-CKD agent); whether a drug delivery system is utilized; and whether the anti-CKD agent is administered as part of a drug combination. Thus, the dosage regimen actually employed can vary widely, and can differ from the dosage regimen set forth above.

In a further aspect, the disclosure provides a composition suitable for preventing CKD in a feline susceptible to developing renal insufficiency. The composition comprises from about from about 33 to about 38%, or 28 to about 35% protein on a dry matter basis wherein the protein comprises at least about 75% vegetable protein.

Since CKD is a terminal disease in felines, it is important to prevent a decrease in renal function in a feline that does not shows clinical signs of CKD. Such a feline is one which has not been diagnosed with CKD, but is at risk to develop CKD due to, for example, advanced age, genetic predisposition, or disease(s) or medication(s) that can damage the feline's kidney(s). Preventing CKD includes reducing the risk of, delaying the onset of, and/or keeping a feline from developing CKD. Thus, feeding a composition of the invention for preventing CKD can reduce a feline's risk of developing CKD. In addition, feeding such a composition can slow down the progression of loss of a feline's kidneys' built-in reserve, thereby delaying the onset of the later phases of CKD.

The composition for preventing CKD is suitable for any feline which is susceptible to develop CKD. In some embodiments, the feline is a companion feline. A companion feline can be a feline kept as a pet. A companion feline can also be a feline from a widely domesticated species, for example, cats (*Felis domesticus*) regardless of whether or not it is kept as a pet. In some embodiments, the feline is an adult feline. An adult feline is a feline of any age after juvenile growth and development has been completed, including senior and geriatric felines. For example, an adult cat typically is one that is from about one year old through the remainder of its life. A senior feline is one of an age at which it is at a risk for suffering from an age-related disease regardless of whether or not the feline shows obvious physical or behavioral signs of aging. For example, a senior cat typically is a cat from about seven to about eleven years old. A geriatric feline is a feline showing signs of aging. For example, a geriatric cat typically is a cat of about twelve years of age and beyond.

The composition for preventing CKD in a feline comprises from about 33 to about 38%, or from about 28 to about 35% protein. Commercial adult cat foods, for example, typically comprise from about 35 to about 45% protein on a dry matter basis. Thus, a composition of the disclosure for preventing CKD may comprise less protein than most commercially available adult cat foods.

In some embodiments, the composition for preventing CKD comprises from about 28 to about 31%, or from about 33 to about 38% protein on a dry matter basis. In some such embodiments, the composition comprises from about 28 to about 30%, or from about 33 to about 34% protein; and in other such embodiments, the composition comprises from about 29 to about 31 % protein, or from about 33.5 to about 35% protein. In yet other such embodiments, the composition comprises from about 28 or about 28.5 to about 29, about 29.5, about 30, about 30.5, or about 31 % protein. In another embodiment, the composition comprises from about 33 or about 33.5 to about 34, about 34.5, or about 35% protein.

In other embodiments, the composition for preventing CKD comprises from about 31 to about 35%, or from about 35 to about 38% protein. In some such embodiments, the composition comprises from about 32 to about 34% protein, or from about 36 to about 37% protein. In other embodiments, the composition comprises from about 32 to about 35% protein, or from about 36 to about 38% protein. In other embodiments, the composition comprises from about 33 to about 35%, or from about 34 to about 38% protein. In other embodiments, the composition comprises from about 31, about 32, or about 33 to about 34 or about 35% protein. In another embodiment, the composition comprises from about 35, about 36, or about 36.5 to about 37 or 38% protein. In further embodiments, the composition comprises from about 31 to about 32, about 33, about 34, or about 35% protein. In another embodiment, the composition comprises from about 35 to about 36, 37, or about 38% protein.

Dietary protein restriction is beneficial for preventing and treating CKD. Excessive protein is catabolized to urea and other nitrogenous compounds normally excreted by the kidneys. Decreased renal function leads to accumulation of these compounds. On the other hand, endogenous proteins will be degraded if amino acid intake is insufficient to maintain nitrogen balance. Thus, a composition of the invention for preventing and/or treating CKD can facilitate achieving nitrogen balance, and can help limit the accumulation of waste products by decreasing protein intake.

Because felines are carnivores, feline foods typically are formulated to comprise mostly animal and fish protein. However, some characteristics of vegetarian diets are desirable for animals with CKD (e.g., lower amount of protein, lower bioavailability of phosphorus, lower amount of sulfur-containing amino acids thereby reducing diet acidity). Thus, the protein in a composition of the present invention for preventing CKD comprises at least about 75% vegetable protein *(i.e.,* a composition of the disclosure for preventing CKD comprises from about 33 to about 38%, or from about 28 to about 35% protein wherein the protein present in the composition comprises at least about 75% vegetable protein). In some such embodiments, the composition comprises at least about 80% vegetable protein. In other embodiments, the composition comprises at least about 85% vegetable protein. In other such embodiments, the protein comprises at least about 90% vegetable protein. And in other such embodiments, the protein comprises at least about 95% vegetable protein (*i*.*e*., from at least about 95 up to about 100% vegetable protein).

Thus, in some embodiments, the composition for preventing CKD comprises from about 28 to about 31% protein wherein the protein comprises at least about 75% vegetable protein. In some such embodiments, the composition comprises from about 26 to about 30%, or from about 28 to about 30% protein wherein the protein comprises at least about 75, at least about 80, at least about 85, at least about 90, or at least about 95% vegetable protein. In other such embodiments, the composition comprises from about 29 to about 31% protein wherein the protein comprises at least about 75, at least about 80, at least about 85, at least about 90, or at least about 95% vegetable protein. In yet other such embodiments, the composition comprises from about 28 or about 28.5 to about 29, about 29.5, about 30, about 30.5, or about 31% protein wherein the protein comprises at least about 75, at least about 80, at least about 85, at least about 90, or at least about 95% vegetable protein.

In other embodiments, the composition for preventing CKD comprises from about 31 to about 35% protein wherein the protein comprises at least about 75% vegetable protein. In some such embodiments, the composition comprises from about 31, about 32, or about 33 to about 34 or about 35% protein wherein the protein comprises at least about 75, at least about 80, at least about 85, at least about 90, or at least about 95% vegetable protein. In other embodiments, the composition comprises from about 31 to about 32, about 33, about 34, or about 35% protein, wherein the protein comprises at least about 75, at least about 80, at least about 85, at least about 90, or at least about 95% vegetable protein. In yet other embodiments, the composition comprises from about 28 or about 28.5 to about 29, about 29.5, or about 30% protein, wherein the protein comprises at least about 75, at least about 80, at least about 85, at least about 90, or at least about 95% vegetable protein.

As contemplated herein, vegetable protein suitable for preparing compositions of the invention include, but are not limited to, potato concentrate, soy concentrate, soy protein isolate, soybean meal, corn gluten meal, rice protein isolate, pea protein concentrate, wheat protein concentrate, and wheat protein isolate. Vegetable protein may be isolated from any portion of a plant, isolated from more than one portion of a plant, and isolated from more than one plant by methods known by those of skill in the art. Vegetable protein may also be concentrated by methods known by those of skill in the art.

Secondary hyperparathyroidism and phosphorus retention have been incriminated as causes of CKD. Therefore, reduction of phosphorus in a feline's diet can be beneficial for preventing and/or treating CKD. Thus, in some embodiments, the composition of the disclosure for preventing CKD in a feline further comprises from about 0.45 to about 0.8%, from 0.51 to about 0.80%, or from about 0.51 to about 0.70% phosphorus on a dry matter basis. In some such embodiments, the composition comprises about 0.45 to about 0.6% phosphorus or from about 0.51 to about 0.65%, for example, from about 0.45 to about 0.55% or from about 0.45 to about 0.5% phosphorus, or from about 0.51 to about 0.60 or from about 0.51 to about 0.55% phosphorus. In other such embodiments, the composition comprises from about 0.6 or about 0.65 to about 0.8% phosphorus, for example, from about 0.7 to about 0.8% or from about 0.6 or 0.65 to about 0.75% phosphorus. The term "phosphorus" means the phosphorus atom without reference to its molecular or ionic form.

When dietary sodium intake changes, fractional excretion of sodium must change as well to maintain sodium balance. Felines with CKD can only vary sodium excretion over a limited range, which narrows progressively as glomerular filtration rate declines. Thus, such felines cannot tolerate excessively high or excessively low dietary sodium intake. Feeding a feline a composition of the disclosure for preventing and/or treating CKD can help maintain optimal sodium balance. Thus, in some embodiments, the composition of the disclosure for preventing CKD in a feline further comprises from about 0.20 or 0.25 to about 0.40%, or from about 0.2 to about 0.35% sodium on a dry matter basis. In some such embodiments, the composition comprises from about 0.2 to about 0.25 or 0.30% sodium. In other such embodiments, the composition comprises from about 0.25 to about 0.3 or 0.35% sodium. The term "sodium" means the sodium atom without reference to its molecular or ionic form.

Normally, the majority of ingested potassium is excreted in the urine, with the remainder excreted in feces. The proportion of potassium excreted in feces increases in felines with CKD. Such felines typically also have disorders in potassium homeostasis. Feeding such felines a composition of the disclosure for preventing and/or treating CKD can help maintain optimal potassium balance. Thus, in some embodiments, the composition of the invention for use in preventing CKD in a feline further comprises from about 0.75 to about 1% potassium on a dry matter basis. In some such embodiments, the composition comprises from about 0.8 to about 1% potassium. In other such embodiments, the composition comprises from about 0.75 to about 0.9% potassium. The term "potassium" means the potassium atom without reference to its molecular or ionic form.

In some embodiments, the composition of the disclosure for preventing CKD further comprises any two or more of phosphorus, sodium, and potassium in any combination of the amounts discussed above.

In another aspect, the disclosure provides a composition suitable for treating CKD in a feline. The composition comprises from about 26 to about 30%, or about 28 to about 30% protein on a dry matter basis wherein the protein comprises at least about 75% vegetable protein.

Treatment can help a feline with CKD to live a healthier, longer, and more active life. Treating CKD includes ameliorating and or suppressing CKD, which may include, e.g., slowing the decrease in renal function or decrease in glomerular filtration rate of an animal. One skilled in the art can diagnose CKD (as well as distinguish it from other diseases, for example, a kidney tumor, diabetes, and hyperthyroidism) by utilizing blood and urine tests as well as, for example, radiography, ultrasound, renal biopsy, and/or palpation. In addition to other symptoms, felines with CKD typically have an elevated serum creatinine and/or BUN. A composition for treating CKD of the disclosure is suitable for any feline with CKD. Examples of felines that can be treated with a composition of the disclosure are discussed above in the context of the compositions of the invention for use in preventing CKD.

In another embodiment, the composition comprises from about 28 to about 29% protein, and in other such embodiments, the composition comprises from about 29 to about 30% protein. In yet other such embodiments, the composition comprises from about 28 or about 28.5 to about 29, about 29.5, or about 30% protein. The protein in the composition for treating CKD comprises at least about 75% vegetable protein *(i.e.,* a composition of the present disclosure for treating CKD in a feline comprises from about 26 to about 30%, or from about 28 to about 30% protein wherein the protein comprises at least about 75% vegetable protein). In some such embodiments, the composition comprises at least about 80% vegetable protein. In other such embodiments, the composition comprises at least about 85% vegetable protein. In other such embodiments, the protein comprises at least about 90% vegetable protein. In other such embodiments, the protein comprises at least about 95% vegetable protein *(i.e.,* from at least about 95 up to about 100% vegetable protein). In some embodiments, the composition for treating CKD in a feline comprises from about 28 or about 28.5 to about 29, about 29.5, or about 30% protein wherein the protein comprises at least about 75, at least about 80, at least about 85, at least about 90, or at least about 95% vegetable protein.

In some embodiments, the composition for treating CKD further comprises from about 0.20 to about 0.50%, or from about 0.45 to about 0.6% phosphorus on a dry matter basis. In some such embodiments, the composition comprises from about 0.20 to about 0.45, or from about 0.45 to about 0.55% phosphorus; and in other such embodiments from about 0.20 to about 0.40, or from about 0.45 to about 0.5% phosphorus.

In some embodiments, the composition for treating CKD further comprises from about 0.2 to about 0.25%, or from about 0.23 to about 0.30% sodium on a dry matter basis.

In some embodiments, the composition for treating CKD further comprises from about 0.75 to about 1% potassium on a dry matter basis. In some such embodiments, the composition comprises from about 0.7 to about 0.9% potassium; and in other such embodiments from about 0.75 to about 0.85% potassium.

In some embodiments, the composition for treating CKD further comprises any two or more of phosphorus, sodium, and potassium in any combination of the amounts discussed above.

In some embodiments, the compositions of the disclosure for preventing and/or treating CKD comprise a food composition (*i.e.,* the compositions comprise one or more food compositions of the present disclosure). In some embodiments, the compositions meet or are below the AAFCO's minimum nutrient level requirements for reproduction or maintenance. It is understood by those of skill in the art that compositions affecting CKD may be below AAFCO recommendations for phosphorus and protein levels. In some embodiments, the food compositions comprise a dry food. In some embodiments, the food compositions comprise a semi-moist food. In some embodiments, the food compositions comprise a moist food. In some embodiments, the food compositions comprise a treat, snack, supplement, or partially or fully edible toy. In some embodiments, the compositions comprise a mixture of one or more foods.

In a further aspect, the disclosure provides a kit comprising a composition of the invention. The kit is suitable for preventing and/or treating CKD in a feline. In some embodiments, the kit further comprises an anti-CKD agent (*i*.*e*., one or more anti-CKD agents). In some embodiments, the kit further comprises instructions for one or more of (1) feeding the composition to a feline, (2) administering an anti-CKD agent to the feline in conjunction with feeding the feline the composition, (3) preventing CKD in a feline by feeding the feline the composition, (4) treating CKD in a feline by feeding the feline the composition, (5) preventing CKD in a feline by administering to the feline an anti-CKD agent in conjunction with feeding the feline the composition, and (6) treating CKD in a feline by administering to the feline an anti-CKD agent in conjunction with feeding the feline the composition.

In some embodiments, the kit comprises two or more ingredients that, when combined together and optionally with additional ingredients that are or are not a part of the kit, yield a composition of the present disclosure. If such additional ingredients are to be used, the kit provides instructions about these ingredients. In some embodiments, the kit further comprises an anti-CKD agent. In some embodiments, the kit further comprises instructions for one or more of (1) preparing a composition of the disclosure for preventing CKD in a feline by combining the ingredients, (2) preparing a composition of the disclosure for treating CKD in a feline by combining the ingredients, (3) feeding a feline a composition of the disclosure for preventing CKD, (4) feeding a feline a composition of the disclosure for treating CKD, (5) administering an anti-CKD agent to a feline in conjunction with feeding the feline a composition of the disclosure for preventing CKD, (6) administering an anti-CKD agent to a feline in conjunction with feeding the feline a composition of the disclosure for treating CKD, (7) preventing CKD in a feline by feeding the feline a composition of the disclosure for preventing CKD, (8) treating CKD in a feline by feeding the feline a composition of the disclosure for treating CKD, (9) preventing CKD in a feline by administering to the feline an anti-CKD agent in conjunction with feeding the feline a composition of the disclosure for preventing CKD, and (10) treating CKD in a feline by administering to the feline an anti-CKD agent in conjunction with feeding the feline a composition of the disclosure for treating CKD.

In some embodiments, the kit comprises in separate containers in a single package or in separate containers in a virtual package, as appropriate for the kit component, a composition of the disclosure or two or more ingredients, that, when combined together and optionally with additional ingredients that are or are not a part of the kit, yield a composition of the disclosure, and instructions for one or more of (1) preparing a composition of the disclosure for preventing CKD in a feline by combining the ingredients, (2) preparing a composition of the disclosure for treating CKD in a feline by combining the ingredients, (3) feeding a feline a composition of the disclosure to prevent CKD, (4) feeding a feline a composition of the disclosure to treat CKD, (5) administering an anti-CKD agent to a feline in conjunction with feeding the feline a composition of the disclosure to prevent CKD, (6) administering an anti-CKD agent to a feline in conjunction with feeding the feline a composition of the disclosure to treat CKD, (7) preventing CKD in a feline by feeding the feline a composition of the disclosure for preventing CKD, (8) treating CKD in a feline by feeding the feline a composition of the disclosure for treating CKD, (9) preventing CKD in a feline by administering to the feline an anti-CKD agent in conjunction with feeding the feline a composition of the disclosure for preventing CKD, and (10) treating CKD in a feline by administering to the feline an anti-CKD agent in conjunction with feeding the feline a composition of the disclosure for treating CKD.

The term "single package" generally means that the components of a kit are physically associated in or with one or more containers and considered as a unit of manufacture, distribution, sale, or use. Containers include, for example, bags, boxes, bottles, shrink wrap packages, stapled or otherwise fixed components, and combinations thereof. A single package can be, for example, containers or individual food compositions physically associated such that they are considered a unit for manufacture, distribution, sale, or use. The term "virtual package" generally means that the components of a kit are associated by directions on one or more physical or virtual kit components instructing the user how to obtain additional components, e.g., in a bag containing one component and directions instructing the user to go to a website, contact a recorded message, view a visual message, or contact a caregiver to obtain instructions on how to use the kit. When the kit comprises a virtual package, the kit is limited to instructions in a virtual environment with one or more physical kit components.

In some embodiments, the kit is suitable for preventing chronic kidney disease in a feline and comprises in separate containers in a single package or in separate containers in a virtual package. as appropriate, a composition that comprises from about 33 to about 38%, or from about 28 to about 35% protein on a dry matter basis wherein the protein comprises at least about 75% vegetable protein, or two or more ingredients that, when combined together and. optionally, with additional ingredients that are or are not a part of the kit, yield a composition that comprises from about 33 to about 38%, or from about 28 to about 35% protein wherein the protein comprises at least about 75% vegetable protein, and one or more of (1) an anti-chronic kidney disease agent, (2) instructions for preparing the composition, (3) instructions for feeding the composition to a feline. (4) instructions for preventing chronic kidney disease in a feline by feeding the feline the composition, and (5) instructions for preventing chronic kidney disease in a feline by administering to the feline an anti-chronic kidney disease agent in conjunction with feeding the feline the composition. In some such embodiments, the kit comprises a composition that comprises from about 31 to about 35% protein, or two or more ingredients that, when combined together and, optionally, with additional ingredients that are or are not a part of the kit, yield a composition that comprises from about 31 to about 35% protein.

In other embodiments, the kit is suitable for treating chronic kidney disease in a feline and comprises in separate containers in a single package or in separate containers in a virtual package, as appropriate, a composition that comprises from about 26 to about 30%, or from about 28 to about 30% protein wherein the protein comprises at least about 75% vegetable protein, or two or more ingredients that. when combined together and, optionally, with additional ingredients that are or are not a part of the kit, yield a composition that comprises from about 26 to about 30%, or from about 28 to about 30% protein wherein the protein comprises at least about 75% vegetable protein, and one or more of (1) an anti-chronic kidney disease agent. (2) instructions for preparing the composition, (3) instructions for feeding the composition to a feline, (4) instructions for treating chronic kidney disease in a feline by feeding the feline the composition, and (5) instructions for treating chronic kidney disease in a feline by administering to the feline an anti-chronic kidney disease agent in conjunction with feeding the feline the composition.

In another aspect, the disclosure provides a means for communicating information about or instructions for one or more of (1) feeding a feline a composition of the disclosure to prevent CKD, (2) feeding a feline a composition of the disclosure to treat CKD, (3) administering an anti-CKD agent to a feline in conjunction with feeding the feline a composition of the disclosure to prevent CKD, (4) administering an anti-CKD agent to a feline in conjunction with feeding the feline a composition of the disclosure to treat CKD, (5) preventing CKD in a feline by feeding the feline a composition of the disclosure for preventing CKD, (6) treating CKD in a feline by feeding the feline a composition of the disclosure for treating CKD, (7) preventing CKD in a feline by administering to the feline an anti-CKD agent in conjunction with feeding the feline a composition of the disclosure for preventing CKD, (8) treating CKD in a feline by administering to the feline an anti-CKD agent in conjunction with feeding the feline a composition of the disclosure for treating CKD, (9) using a kit of the disclosure to prevent CKD, and (10) using a kit of the disclosure to treat CKD. The communication means comprise, for example, a document, digital storage media, optical storage media, audio presentation, or visual display containing the information or instructions. Preferably, the communication means is a displayed web site or a brochure, product label, package insert, advertisement, or visual display containing such information or instructions. Useful information or instructions include, for example, (1) information and instructions how to use a composition, method, or kit of the disclosure and (2) contact information for animal caregivers if they have a question about the invention and its uses.

In some embodiments, the present disclosure provides a means for communicating information about or instructions for one or more of (1) preventing chronic kidney disease in a feline by feeding the feline a composition of the disclosure that comprises from about 33 to about 38%, or from about 28 to about 35% protein wherein the protein comprises at least about 75% vegetable protein, (2) preventing chronic kidney disease in a feline by administering to the feline an anti-chronic kidney disease agent in conjunction with feeding the feline a composition of the disclosure that comprises from about 33 to about 38%, or from about 28 to about 35% protein wherein the protein comprises at least about 75% vegetable protein, and (3) using a kit of the disclosure for preventing CKD in a feline that comprises a composition comprising from about 33 to about 38%, or from about 28 to about 35% protein wherein the protein comprises at least about 75% vegetable protein or two or more ingredients that, when combined together and, optionally, with additional ingredients, yield a composition that comprises from about 33 to about 38%, or from about 28 to about 35% protein wherein the protein comprises at least about 75% vegetable protein. The means comprise a document, digital storage media, audio presentation, or visual display containing the information or instructions.

In other embodiments, the present disclosure provides a means for communicating information about or instructions for one or more of (1) treating chronic kidney disease in a feline by feeding the feline a composition of the disclosure that comprises from about 26 to about 30%, or from about 28 to about 30% protein on a dry matter basis wherein the protein comprises at least about 75% vegetable protein, (2) treating chronic kidney disease in a feline by administering to the feline an anti-chronic kidney disease agent in conjunction with feeding the feline a composition of the disclosure that comprises from about 26 to about 30%, or from about 28 to about 30% protein on a dry matter basis wherein the protein comprises at least about 75% vegetable protein, and (3) using a kit of the disclosure for treating CKD in a feline that comprises a composition comprising from about 26 to about 30%, or from about 28 to about 30% protein wherein the protein comprises at least about 75% vegetable protein or two or more ingredients that, when combined together and, optionally, with additional ingredients, yield a composition that comprises from about 26 to about 30%, or from about 28 to about 30% protein wherein the protein comprises at least about 75% vegetable protein. The communication means comprise a document, digital storage media, audio presentation, or visual display containing the information or instructions.

In another aspect, the disclosure provides for a use of a composition of the disclosure to prepare a medicament suitable for preventing feline CKD. As described herein, the composition generally comprises from about 33 to about 38%, or from about 28 to about 35% protein on a dry matter basis wherein the protein comprises at least about 75% vegetable protein. In some embodiments, the composition comprises from about 33 to about 35%, or from about 28 to about 31% protein wherein the protein present in the composition comprises at least about 75% vegetable protein. In some such embodiments, the composition comprises from about 33 to about 34%, or from about 26 to about 30%, or from about 28 to about 30% protein wherein the protein comprises at least about 75, at least about 80, at least about 85, at least about 90, or at least about 95% vegetable protein. In other such embodiments, the composition comprises from about 34 to about 35%, or from about 29 to about 31% protein wherein the protein comprises at least about 75, at least about 80, at least about 85, at least about 90, or at least about 95% vegetable protein. In yet other such embodiments, the composition comprises from about 28 or about 28.5 to about 29, about 29.5, about 30, about 30.5, or about 31 % protein wherein the protein comprises at least about 75, at least about 80, at least about 85, at least about 90, or at least about 95% vegetable protein. In another embodiment, the composition comprises from about 33 or about 33.5 to about 34, about 34.5, or about 35% protein wherein the protein comprises at least about 75, at least about 80, at least about 90, or at least about 95% vegetable protein. In other embodiments, the composition comprises from about 31 to about 35% protein wherein the protein comprises at least about 75% vegetable protein. In some such embodiments, the composition comprises from about 31, about 32, or about 33 to about 34 or about 35% protein wherein the protein comprises at least about 75, at least about 80, at least about 85, at least about 90, or at least about 95% vegetable protein. In another embodiment, the composition comprises from about 35, about 36, or about 36.5 to about 37 or about 38% protein wherein the protein comprises at least about 75, at least about 80, at least about 85, at least about 90, or at least about 95% vegetable protein. In other such embodiments, the composition comprises from about 31 to about 32, about 33, about 34, or about 35% protein wherein the protein comprises at least about 75, at least about 80, at least about 85, at least about 90, or at least about 95% vegetable protein. In some embodiments, the composition further comprises one or more of sodium, phosphorus, and potassium in the amounts discussed above. In another embodiment, the composition comprises from about 35 to about 36, about 36.5, about 37, or about 38% protein wherein the protein comprises at least about 75, at least about 80, at least about 85, at least about 90, or at least about 95% vegetable protein. And in some embodiments, the composition comprises a food composition.

In another aspect, the disclosure provides for a use of a composition of the disclosure to prepare a medicament suitable for treating feline CKD. As described herein, the composition generally comprises from about 26 to about 30%, or from about 28 to about 30% protein on a dry matter basis wherein the protein comprises at least about 75% vegetable protein. In some embodiments, the composition comprises from about 28 or about 28.5 to about 29, about 29.5, or about 30% protein wherein the protein comprises at least about 75, at least about 80, at least about 85, at least about 90, or at least about 95% vegetable protein. In some embodiments, the composition comprises from about 26 or about 27 to about 28, about 29, or about 30% protein wherein the protein comprises at least about 75, at least about 80, at least about 85, at least about 90, or at least about 95% vegetable protein. In some embodiments, the composition further comprises one or more of phosphorus, sodium, and potassium in the amounts discussed above. And in some embodiments, the composition comprises a food composition.

Generally, medicaments are prepared by admixing a compound or composition with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to a feline.

In another aspect, the disclosure provides methods for preparing compositions of the disclosure for preventing and/or treating CKD. Such composition can be prepared, for example, by mixing various ingredients (including food compositions) that, when combined, yield a composition of the disclosure. Compositions of the can also be prepared by the methods discussed in, for example, Small Animal Nutrition, pages 127-146 (2000). As discussed above, plant ingredients suitable for preparing compositions of the disclosure include, for example, potato concentrate, soy concentrate, soybean meal, soy protein isolate, corn gluten meal, rice protein isolate, pea protein concentrate, wheat protein concentrate, and wheat protein isolate.

Creatinine levels in an animal may be measured by any method known by those of skill in the art. The compositions of the present disclosure, e.g., compositions to treat or prevent CKD, may also be suitable to lower the creatinine serum levels in an animal. Preferably, the animal is a companion animal, e.g., a feline. The methods of the present disclosure, e.g., to treat or prevent CKD may be suitable to lower the creatinine serum levels in an animal.

For the methods of the present disclosure, preferably, an effective amount is fed to the animal. "An effective amount" refer to that amount of a compound, material or composition as described herein that may be effective to achieve a particular biological result. Such results may include, but are not limited to, the treatment and/or prevention of CKD. An effective amount may be based on several factors, including an animal's ideal weight, the metabolizable energy of the composition, and frequency of feeding the animal compositions of the present disclosure, e.g., once, twice, or three times daily, and other compositions fed to the animal.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. Similarly, the words "comprise", "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, devices, and materials are described herein.

### EXAMPLES

The invention can be further illustrated by the following examples, although it will be understood that the examples are included merely for purposes of illustration and are not intended to limit the scope of the invention unless otherwise specifically indicated.

### Reference Example 1

Reference Example 1 illustrates the effect of the compositions of the disclosure on preventing CKD in healthy cats.

Food A is formulated as a dry cat food meeting the AAFCO's minimum nutrient level requirements for maintenance for cats. It contains soybean meal, corn gluten meal, poultry meal, and pork meat protein isolate as protein ingredients. Food A contains approximately 34% total protein on a dry matter basis; 97% of the protein was from vegetable and 3% from animal. Food A also contains 0.61% phosphorous, and 0.35% sodium.

Food B1 is formulated as a dry cat food meeting AAFCO's minimum nutrient level requirements for maintenance for cats. It contains soybean meal. corn gluten meal, poultry meal, and pork meat protein isolate as protein ingredients. Food B1 contains approximately 34% total protein on a dry matter basis; 77% of the protein was from vegetable. Food B1 also contains 0.60% phosphorous, and 0.33% sodium.

Food B2 is formulated as a wet cat food meeting AAFCO's minimum nutrient level requirements for maintenance for cats. It contains soybean meal, corn gluten meal, pork lungs, chicken, and pork liver as protein ingredients. Food B2 contains approximately 34% total protein on a dry matter basis; 32% of the protein was from vegetable. Food B2 also contains 0.60% phosphorous, and 0.30% sodium.

Food C is formulated as a dry cat food meeting AAFCO's minimum nutrient level requirements for maintenance for cats. It contains corn gluten meal, poultry meal, and pork meat protein isolate as protein ingredients. Food C contains approximately 34% total protein on a dry matter basis; 85% of the protein was from vegetable. Food C also contains 0.62% phosphorous, and 0.35% sodium.

Eight healthy cats are fed food A for 26 weeks. Another eight healthy cats are fed food C for 26 weeks. Six healthy cats are fed food B1 for 26 weeks. And another six healthy cats are fed food B2 for 26 weeks. The serum creatinine levels of all cats is measured at the beginning and at the completion of the study (*i*.*e*., at day 0 and at 26 weeks, respectively) (Boehringer Mannheim automated serum chemistry analyzer). The results are presented in Table 1.

**Table 1**

| Change in Serum Creatinine Levels (Normal cats) | | | | | |
|---|---|---|---|---|---|
| Food | Total protein (% DMB) | % Protein from Vegetable (% DMB) | Creatinine at day 0 (mg/dL) | Creatinine at week 26 (mg/dL) | Creatinine change (mg/dL) |
| Food A | 34 | 97.0 | 1.53 | 1.14 | -0.39 |
| Food B1 | 34 | 76.5 | 1.28 | 1.22 | -0.06 |
| Food B2 | 34 | 32.0 | 1.48 | 1.39 | -0.09 |
| Food C | 34 | 85.0 | 1.46 | 1.10 | -0.36 |

All cats had normal serum creatinine level at the beginning of the study. Serum creatinine levels of approximately 0.8 to approximately 1.8 mg/dL are considered normal. The cats that were fed food B1 had the lowest initial serum creatinine level. A significant reduction in serum creatinine level was observed in the cats fed foods A and C *(i.e.,* the foods with the highest vegetable protein content), particularly when compared to the cats fed food B2 *(i.e.,* the food with the lowest vegetable protein content). No significant reduction in serum creatinine level was observed in the cats fed foods B1 compared to the cats fed food B2.

### Reference Example 2

Reference Example 2 illustrates the effect of the compositions of the disclosure on treating CKD in cats with renal insufficiency and age matched normal cats.

Food D was formulated as a dry cat food meeting the AAFCO's minimum nutrient level requirements for maintenance for cats. It contained approximately 28% total protein on a dry matter basis; 79% of the protein was from vegetable origin.

Six cats diagnosed with renal insufficiency and 6 healthy cats were fed food D for 26 weeks. The serum creatinine and serum phosphorus levels of all cats were at the beginning of the study, 13, and 26 weeks. Data for week 26 is not presented, as n=2 at 26 weeks. The results are presented in Tables 2 and 3.

**Table 2**

| Change in Serum Creatinine Levels | | | |
|---|---|---|---|
| | Creatinine at day 0 (mg/dL) | Creatinine at week 13 (mg/dL) | Creatinine change (mg/dL) |
| CKD cats | 2.46 | 2.15 | -0.31 |
| Healthy cats | 1.24 | 1.03 | -0.21 |

**Table 3**

| Change in Serum Phosphorus Levels | | | |
|---|---|---|---|
| | Phosphorus at day 0 (mg/dL) | Phosphorus at week 26 (mg/dL) | Phosphorus change (mg/dL) |
| CKD cats | 4.79 | 4.46 | -0.33 |
| Healthy cats | 4.92 | 4.64 | -0.28 |

Feeding food D reduced serum creatinine levels in both the healthy cats and the cats with CKD. In addition, feeding food D also resulted in reduction in serum phosphorus level in both the healthy cats and the cats with CKD (phosphorus levels of approximately 3 to approximately 5.6 mg/dL are considered normal).

Unless otherwise stated, all percentages provided herein are weight percentages on a dry matter basis. The term "dry matter basis" (DMB) means that an ingredient's concentration in a composition is measured after removing the moisture from the composition.

In the specification, there have been disclosed typical preferred embodiments of the invention and, although specific terms are employed, they are used in a generic and descriptive sense only and not for purposes of limitation, the scope of the invention being set forth in the claims. It is therefore to be understood that within the scope of the appended claims the invention may be practiced otherwise than as specifically described.

## Claims

1. A composition comprising from 33 to 38% protein, and from 0.6 to 0.8% phosphorus on a dry matter basis, and an anti-chronic kidney disease agent selected from: agents that reduce blood pressure or protein loss in urine, ACE-inhibitors, anabolic steroids, antibiotics, agents that treat anemia, diuretics, agents that control vomiting, and salts thereof, wherein the protein comprises at least 75% vegetable protein, for use in preventing chronic kidney disease in a feline.

2. The composition for use accoding to claim 1 wherein the composition further comprises from 0.25 to 0.35% sodium on a dry matter basis and/or from 0.75 to 1% potassium on a dry matter basis.

3. The composition for use according to claim 1 or claim 2 wherein the feline is a cat.

4. The composition for use according to any preceding claim wherein the composition is a food composition.

5. Use of a composition comprising from 33 to 38% protein, from 0.6 to 0.8% phosphorus on a dry matter basis and an anti-chronic kidney disease agent selected from: agents that reduce blood pressure or protein loss in urine, ACE-inhibitors, anabolic steroids, antibiotics, agents that treat anemia, diuretics, agents that control vomiting, and salts thereof, wherein the protein comprises at least 75% vegetable protein, to prepare a medicament for use in preventing chronic kidney disease in a feline.

## Patentansprüche

1. Zusammensetzung, die von 33 bis 38% Protein und von 0,6 bis 0,8% Phosphor auf einer Trockenmassebasis und ein Mittel gegen chronische Nierenerkrankung umfasst, ausgewählt aus: Mitteln, die Blutdruck oder Proteinverlust im Urin verringern, ACE-Inhibitoren, anabolen Steroiden, Antibiotika, Mitteln, die Anämie behandeln, Diuretika, Mitteln, die Erbrechen kontrollieren, und Salzen davon, wobei das Protein mindestens 75% Pflanzenprotein umfasst, zur Verwendung beim Vorbeugen von chronischer Nierenerkrankung in einem Katzenartigen.

2. Zusammensetzung zur Verwendung nach Anspruch 1, wobei die Zusammensetzung weiterhin von 0,25 bis 0,35% Natrium auf einer Trockenmassebasis und/oder von 0,75 bis 1% Kalium auf einer Trockenmassebasis umfasst.

3. Zusammensetzung zur Verwendung nach Anspruch 1 oder Anspruch 2, wobei der Katzenartige eine Katze ist.

4. Zusammensetzung zur Verwendung nach einem beliebigen voranstehenden Anspruch, wobei die Zusammensetzung eine Nahrungsmittelzusammensetzung ist.

5. Verwendung einer Zusammensetzung, die von 33 bis 38% Protein, von 0,6 bis 0,8% Phosphor auf einer Trockenmassebasis und ein Mittel gegen chronische Nierenerkrankung umfasst, ausgewählt aus: Mitteln, die Blutdruck oder Proteinverlust im Urin verringern, ACE-Inhibitoren, anabolen Steroiden, Antibiotika, Mitteln, die Anämie behandeln, Diuretika, Mitteln, die Erbrechen kontrollieren, und Salzen davon, wobei das Protein mindestens 75% Pflanzenprotein umfasst, um ein Medikament zur Verwendung beim Vorbeugen von chronischer Nierenerkrankung in einem Katzenartigen herzustellen.

## Revendications

1. Composition comprenant de 33 à 38 % de protéine et de 0,6 à 0,8 % de phosphore sur une base de matière sèche, et un agent anti-maladie rénale chronique choisi parmi : les agents qui réduisent la tension artérielle ou la perte de protéine dans les urines, les inhibiteurs de l'ECA, les stéroïdes anabolisants, les antibiotiques, les agents qui traitent l'anémie, les diurétiques, les agents qui contrôlent les vomissements et les sels de ceux-ci, dans laquelle la protéine comprend au moins 75 % de protéine végétale, pour une utilisation dans la prévention d'une maladie rénale chronique chez un félin.

2. Composition pour une utilisation selon la revendication 1, dans laquelle la composition comprend en outre de 0,25 à 0,35 % de sodium sur une base de matière sèche et/ou de 0,75 à 1 % de potassium sur une base de matière sèche.

3. Composition pour une utilisation selon la revendication 1 ou la revendication 2, dans laquelle le félin est un chat.

4. Composition pour une utilisation selon l'une quelconque des revendications précédentes, dans laquelle la composition est une composition alimentaire.

5. Utilisation d'une composition comprenant de 33 à 38 % de protéine, de 0,6 à 0,8 % de phosphore sur une base de matière sèche et un agent anti-maladie rénale chronique choisi parmi : les agents qui réduisent la tension artérielle ou la perte de protéine dans l'urine, les inhibiteurs de l'ECA, les stéroïdes anabolisants, les antibiotiques, les agents qui traitent l'anémie, les diurétiques, les agents qui contrôlent les vomissements et les sels de ceux-ci, dans laquelle la protéine comprend au moins 75 % de protéine végétale, pour préparer un médicament pour une utilisation dans la prévention d'une maladie rénale chronique chez un félin.
